# EUROPÄISCHE PATENTANMELDUNG

(11) **EP 3 037 075 A2**
(43) Veröffentlichungstag der Anmeldung: **29.06.2016**
(21) Anmeldenummer: 15200015.4
(22) Anmeldetag: 15.12.2015
(51) Int. Cl.: A61F 5/01

(54) **ORTHESE MIT INKLINATIONSVERSTELLEINRICHTUNG**

(30) Priorität: 23.12.2014 DE 102014019715
(71) Anmelder: Albrecht GmbH, 83071 Stephanskirchen (DE)
(72) Erfinder: KAHLES, Martin, 83128 Halfing (DE); OPAHLE, Hans-Georg, 83024 Rosenheim (DE)
(74) Vertreter: Bauer, Friedrich

(57) **Zusammenfassung**

Eine Orthese, insbesondere Knieorthese, umfasst ein Paar Schienenarme (3, 4), die jeweils einen schwenkbar gelagerten ersten Schienenarmabschnitt (25) und einen an dem Körper eines Patienten befestigbaren zweiten Schienenarmabschnitt (26) aufweist. Der zweite Schienenarmabschnitt (26) zumindest eines Schienenarms (3, 4) ist derart neigungsverstellbar und in Längsrichtung des Schienenarms 3, 4 verschiebbar am ersten Schienenarmabschnitt (25) gehaltert und zwangsgeführt, dass ein Verschwenken der Schienenarme (3, 4) in Flexions- oder Extensionsrichtung sowohl mit einer Verstellung in der medialen oder lateralen Inklination des zweiten Schienenarmabschnitts (26) als auch mit einer Längenänderung des Schienenarms (3, 4) gekoppelt ist.

## Beschreibung

Die Erfindung betrifft eine Orthese, insbesondere Knieorthese, mit einem Paar Schienenarmen, die jeweils einen schwenkbar gelagerten ersten Schienenarmabschnitt und einen an dem Körper eines Patienten befestigbaren zweiten Schienenarmabschnitt aufweisen, einer Gelenkeinrichtung zum Verschwenken der Schienenarme in Flexions- und Extensionsrichtung, und einer Inklinationsverstelleinrichtung zum Verstellen der medialen oder lateralen Inklination des zweiten Schienenarmabschnitts zumindest eines Schienenarms relativ zur Gelenkeinrichtung, gemäß dem Oberbegriff des Anspruches 1.

Orthesen dieser Art werden insbesondere bei Patienten eingesetzt, die an einer medialen oder lateralen Kniegelenksarthrose (Gonarthrose) leiden. Bei einer medialen Kniegelenksarthrose ist das mediale (innere) Kompartment des Femorotibialgelenks betroffen, während bei einer lateralen Kniegelenksarthrose eine Arthrose des lateralen (äußeren) femorotibialen Kompartments vorliegt. Eine mediale Kniegelenksarthrose ist insbesondere bei Personen verbreitet, die eine O-Bein-Fehlstellung haben. In diesem Fall spricht man von einer Varus-Gonarthrose. Eine laterale Kniegelenksarthrose kommt häufig bei Patienten vor, die eine X-Bein-Fehlstellung haben. In diesem Fall spricht man von einer Valgus-Gonarthrose.

Bei Patienten mit einer medialen oder lateralen Gonarthrose ist in vielen Fällen die Anwendung von Orthesen in Ergänzung zu medikamentösen, physiotherapeutischen und/ oder operativen Therapien vorteilhaft. Es ist in vielen Fällen möglich, durch geeignete Orthesen Belastungsschmerzen des Betroffenen zu lindern und die Gelenkfunktion zu verbessern. Weiterhin können Patienten mit vollständig arthrotisch zerstörten Kniegelenken und einer Varus- oder Valgus-Instabilität durch derartige Orthesen wieder begrenzt gehfähig werden. Derartige Orthesen bieten nichtoperativ die Möglichkeit, extern einen Valgus- bzw. Varus-Stress auszuüben und somit das betroffene Kompartment zu entlasten. Weiterhin haben sich derartige Orthesen auch postoperativ nach Operationen zum Aufbau des Gelenkknorpels oder Meniskusrekonstruktionen bewährt. Auch präoperativ vor einer valgisierenden Korrekturosteotomie des Kniegelenkes kann durch derartige Orthesen bis zu einem gewissen Grad der zu erwartende Behandlungserfolg simuliert werden.

Um auf das Kniegelenk eine medial oder lateral gerichtete Kraft auszuüben, ist aus der WO 94/15555 A1 bereits eine Orthese bekannt, bei der die verschwenkbaren Schienenarme jeweils zweigeteilt sind. Die Schienenarme weisen dort jeweils einen ersten Schienenarmabschnitt auf, der schwenkbar an einer Trägerplatte des Schienengelenks gelagert ist, und einen zweiten Schienenarmabschnitt, der mittels Einstellschrauben in einer bestimmten medialen oder lateralen Winkelposition am ersten Schienenarmabschnitt und mittels Manschetten am Ober- bzw. Unterschenkel des Patienten befestigt werden kann. Nachteilig ist hierbei, dass nach dem Einstellen der medialen oder lateralen Inklination (Schrägstellung) der zweiten Schienenarmabschnitte die mediale bzw. laterale Kraft fortwährend auf das Kniegelenk bzw. den Ober- und Unterschenkel einwirkt, was von den Patienten als unangenehm empfunden wird und auch zu unerwünschten Druckstellen, Traumata und Durchblutungsstörungen an denjenigen Stellen führen kann, an denen die Schienenarme am Körper befestigt sind. Ferner wird durch die dortige Orthese die gewünschte Entlastung des geschädigten Kniegelenkkompartments nicht in dem gewünschten Ausmaß erzielt.

Aus der US 7 500 957 B2 ist eine Orthese gemäß dem Oberbegriff des Anspruchs 1 bekannt, bei der die äußeren Schienenarmabschnitte mittels Zusatzgelenken in medialer oder lateraler Richtung schwenkbar an inneren Schienenarmabschnitten gelagert sind. Die äußeren Schienenarmabschnitte werden dort mittels einer Nockenanordnung aktiv in Richtung des Beins geneigt, wenn das Knie in seine volle Extensionsstellung bewegt wird. Es hat sich jedoch gezeigt, dass auch durch diese Orthese nicht der gewünschte Entlastungseffekt für das Kniegelenk erreicht wird.

Der Erfindung liegt daher die Aufgabe zugrunde, eine Orthese der eingangs genannten Art zu schaffen, die in medizinischer und ergonomischer Hinsicht für mediale und laterale Arthrosen besonders geeignet ist und eine besonders wirkungsvolle Entlastung des geschädigten Gelenkkompartments ermöglicht.

Diese Aufgabe wird erfindungsgemäß durch eine Orthese mit den Merkmalen des Anspruchs 1 gelöst. Vorteilhafte Ausführungsformen der Erfindung sind in den weiteren Ansprüchen beschrieben.

Bei der erfindungsgemäßen Orthese ist der am Körper des Patienten befestigbare zweite Schienenarmabschnitt zumindest eines Schienenarms derart neigungsverstellbar und in Längsrichtung des Schienenarms verschiebbar am ersten Schienenarmabschnitt gehaltert und zwangsgeführt, dass ein Verschwenken des Schienenarms in Flexions- oder Extensionsrichtung sowohl mit einer Verstellung der medialen oder lateralen Inklination des zweiten Schienenarmabschnitts als auch mit einer Längenänderung des Schienenarms gekoppelt ist.

Die erfindungsgemäße Orthese ermöglicht im Unterschied zu bekannten Orthesen, bei denen die mediale oder laterale Inklination des zweiten Schienenarmabschnitts über den gesamten Schwenkbereich der Schienenarme gleich bleibt, eine Veränderung der Inklination in Abhängigkeit der Schwenkstellung der Schienenarme. Hierdurch ist es insbesondere möglich, die Inklination zu minimieren, wenn das Gelenk, insbesondere Kniegelenk, stark angewinkelt ist, beispielsweise wenn Unter- und Oberschenkel zueinander einen Winkel von 90° oder weniger einnehmen, wie dies beim Sitzen der Fall ist. In diesem stark abgewinkelten Zustand des Kniegelenks und damit auch der beiden Schienenarme würden auch bei starker Inklination der Schienenarme die von den Schienenarmen ausgeübten medialen bzw. lateralen Kräfte nicht in der gewünschten Weise auf das Kniegelenk einwirken, so dass die Minimierung der Inklination in der stark abgewinkelten Stellung der Schienenarme keinen negativen Einfluss hinsichtlich der Entlastung des geschädigten Kniegelenkkompartments hat. Die gewünschte Entlastung des geschädigten Kniegelenkkompartments kann dagegen besonders wirkungsvoll bewerkstelligt werden, wenn das Knie gestreckt ist oder sich zumindest nahe seiner Streckstellung befindet. Mit Hilfe der erfindungsgemäßen Orthese ist es daher möglich, die medialen und lateralen Druck- und Zugkräfte der Orthese in der abgewinkelten Stellung des Kniegelenks zumindest weitgehend zu reduzieren, während sie in der gestreckten Stellung des Knies in vollem Umfang auf den Körper des Patienten aufgebracht werden. Dies führt zu einem wesentlich verbesserten Tragekomfort und beugt Traumata und Durchblutungsstörungen in denjenigen Bereichen des Körpers vor, an denen die Schienenarme anliegen bzw. befestigt sind. Insbesondere wird der Patient entlastet, wenn der Patient eine sitzende Stellung einnimmt.

Von besonderem Vorteil ist weiterhin, dass bei einem Verschwenken der Orthese in Flexions- oder Extensionsrichtung nicht nur eine aktive, dynamische Neigungsverstellung in medialer oder lateraler Richtung erfolgt, sondern gleichzeitig auch eine vorbestimmte Längenverstellung des Schienenarms. Die erfindungsgemäße Orthese ist insbesondere derart ausgebildet, dass dann, wenn ein Knie in seine volle Extensionsstellung bewegt wird, sich der zweite Schienenarmabschnitt von der dem betreffenden Schienenarm zugeordneten Schwenkachse und damit von der zentralen Gelenkeinrichtung entfernt, so dass sich der Schienenarm verlängert. Hierdurch wird das Kniegelenk über die am Ober- und Unterschenkel befestigten Manschetten aktiv auseinandergezogen. Dies führt zu einer besonders wirkungsvollen Entlastung des geschädigten Kniegelenkkompartments und begünstigt die durch die mediale oder laterale Inklination der Schienenarme auf das Kniegelenk aufgebrachte Korrekturbewegung in medialer oder lateraler Richtung. Wird das Knie dagegen gebeugt, verkürzen sich vorteilhafterweise die Schienenarme bei gleichzeitiger Verringerung der medialen oder lateralen Inklination.

Gemäß einer vorteilhaften Ausführungsform umfasst die Inklinationsverstelleinrichtung eine Kulissenführung, mit welcher der zweite Schienenarmabschnitt verschiebbar am ersten Schienenarmabschnitt geführt ist. Hierbei kann die Kulissenführung mindestens eine am ersten oder zweiten Schienenarmabschnitt angeordnete, in medialer oder lateraler Richtung gebogene und/oder schräg verlaufende Führungskulisse und mindestens ein am anderen Schienenarmabschnitt angeordnetes, in die Führungskulisse eingreifendes Führungselement umfassen. Derartige Kulissenführungen ermöglichen es insbesondere, die Inklination kontinuierlich und gleichmäßig zu ändern, wenn die Schienenarme in Extensions- oder Flexionsrichtung geschwenkt werden, während gleichzeitig eine stabile und präzise Führung des zweiten Schienenarmabschnitts am ersten Schienenarmabschnitt gewährleistet ist.

Vorteilhafterweise weist der erste oder zweite Schienenarmabschnitt einen U-förmigen Endabschnitt mit gegenüberliegenden Führungsschenkeln auf, in denen jeweils eine Führungskulisse angeordnet ist, wobei der andere, damit zusammenwirkende Schienenarmabschnitt einen zwischen die Führungsschenkel einführbaren Führungsabschnitt mit auf gegenüberliegenden Seiten des Führungsabschnitts angeordneten Führungselementen aufweist. Hierdurch kann eine besonders stabile und exakte Führung des zweiten Schienenarmabschnitts am ersten Schienenarmabschnitt erreicht werden.

Vorteilhafterweise ist das in die Führungskulisse eingreifende Führungselement als länglicher, an die Form der Führungskulisse angepasster Führungssteg ausgebildet. Alternativ hierzu sind jedoch auch andere Arten von Führungselementen denkbar, beispielsweise voneinander beabstandete Führungsbolzen oder -nocken.

Gemäß einer vorteilhaften Ausführungsform weist die Gelenkeinrichtung mindestens eine Trägerplatte auf, an welcher der erste Schienenarmabschnitt um mindestens eine Schwenkachse schwenkbar gelagert ist, wobei eine Verschiebeeinrichtung vorgesehen ist, die einerseits mit der Trägerplatte und andererseits mit dem zweiten Schienenarmabschnitt mindestens eines Schienenarms derart wirkverbunden ist, dass ein Verschwenken des Schienenarms in Flexions- oder Extensionsrichtung sowohl mit einer Verstellung des zweiten Schienenarmabschnitts in medialer oder lateraler Richtung als auch mit einem Entfernen oder Annähern des zweiten Schienenarmabschnitts von der bzw. an die Schwenkachse zwangsgekoppelt ist. Hierdurch kann auf einfache und zuverlässige Weise der zweite Schienenarmabschnitt relativ zum ersten Schienenarmabschnitt und damit zur Gelenkeinrichtung derart bewegt werden, dass sich die Inklination und die Längsposition relativ zur zentralen Gelenkeinrichtung in der gewünschten Weise ändert.

Vorteilhafterweise umfasst die Verschiebeeinrichtung mindestens eine Verbindungsstrebe, die einerseits mit Abstand zur Schwenkachse mit der Trägerplatte und andererseits mit dem zweiten Schienenarmabschnitt mindestens eines Schienenarms verbunden ist, derart, dass sich bei einem Verschwenken des Schienenarms in Flexions- oder Extensionsrichtung der zweite Schienenarmabschnitt relativ zum ersten Schienenarmabschnitt verschiebt.

Die Erfindung wird nachfolgend anhand von Zeichnungen beispielhaft näher erläutert. Es zeigen:
- Figur 1:: eine am Ober- und Unterschenkel angelegte Orthese gemäß der Erfindung zur Entlastung eines Kniegelenks,
- Figur 2a:: ein rechtes Bein mit einer O-Bein-Fehlstellung,
- Figur 2b:: eine schematische Darstellung der Orthese von Figur 1 bei medialer Anordnung am Bein von Figur 2a nach Korrektur der O-Bein-Fehlstellung,
- Figur 2c:: ein rechtes Bein mit einer X-Bein-Fehlstellung,
- Figur 2d:: eine schematische Darstellung der Orthese von Figur 1 bei lateraler Anordnung am Bein von Figur 2c nach Korrektur der X-Bein-Fehlstellung,
- Figur 3:: eine Explosionsdarstellung der erfindungsgemäßen Orthese,
- Figur 4:: eine räumliche Darstellung des ersten Schienenarmabschnitts schräg von oben,
- Figur 5:: einen Längsschnitt durch die erfindungsgemäße Orthese, wobei die Schienenarme verkürzt dargestellt sind und sich in einem Winkel von 180° zueinander befinden,
- Figur 6:: eine Seitenansicht der Orthese von Figur 5, wobei eine äußere Trägerplatte zur Verdeutlichung der darunter liegenden Teile nicht dargestellt ist,
- Figur 7:: eine Vorderansicht der Orthese von Figur 5 und 6,
- Figur 8:: eine Seitenansicht der erfindungsgemäßen Orthese, wobei eine äußere Trägerplatte zur Verdeutlichung der darunter liegenden Teile nicht dargestellt ist,
- Figur 9:: eine Vorderansicht der abgewinkelten Orthese von Figur 8, und
- Figur 10:: eine Vorderansicht der gestreckten Orthese von Figur 7 zur Verdeutlichung der Inklinationsveränderung gegenüber Figur 9.

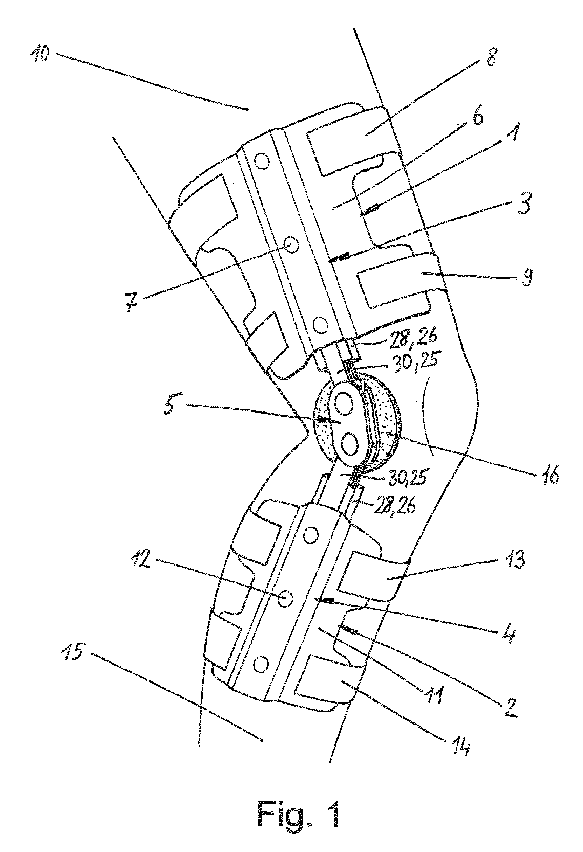

Anhand der Figuren 1 bis 10 wird im Folgenden ein Ausführungsbeispiel der erfindungsgemäßen Orthese in Form einer Knieorthese näher beschrieben. Die erfindungsgemäße Orthese kann jedoch auch für andere Gelenke, beispielsweise das Ellbogengelenk, verwendet werden.

Figur 1 zeigt eine erfindungsgemäße Orthese mit einer oberen Manschette 1, einer unteren Manschette 2, einem ersten Schienenarm 3, einem zweiten Schienenarm 4 und einer zentralen Gelenkeinrichtung 5 zur schwenkbaren Verbindung des ersten und zweiten Schienenarms 3, 4.

Die obere Manschette 1 weist eine obere Manschettenschale 6 auf, an welcher der erste Schienenarm 3 mittels geeigneter Befestigungsmittel, beispielsweise mittels Nieten 7, befestigt ist. An der oberen Manschettenschale 6 ist ein proximales Halteband 8 und ein distales Halteband 9 zur Befestigung der oberen Manschette 1 und damit des ersten Schienenarms 3 an einem Oberschenkel 10 eines Patienten befestigt.

Die untere Manschette 2 ist ähnlich ausgebildet und weist eine untere Manschettenschale 11, an welcher der zweite Schienenarm 4 mittels geeigneter Befestigungsmittel, beispielsweise mittels Nieten 12, befestigt ist, sowie ein proximales Halteband 13 und ein distales Halteband auf, mit denen die untere Manschettenschale 11 und damit der zweite Schienenarm 4 an einem Unterschenkel 15 des Patienten befestigbar ist.

Die Orthese weist zweckmäßigerweise eine geeignete Polsterung auf, um den Tragekomfort zu verbessern. Von dieser Polsterung ist jedoch lediglich ein Polsterkissen 16 dargestellt, das sich auf der dem Bein zugewandten Seite der Gelenkeinrichtung 5 befindet.

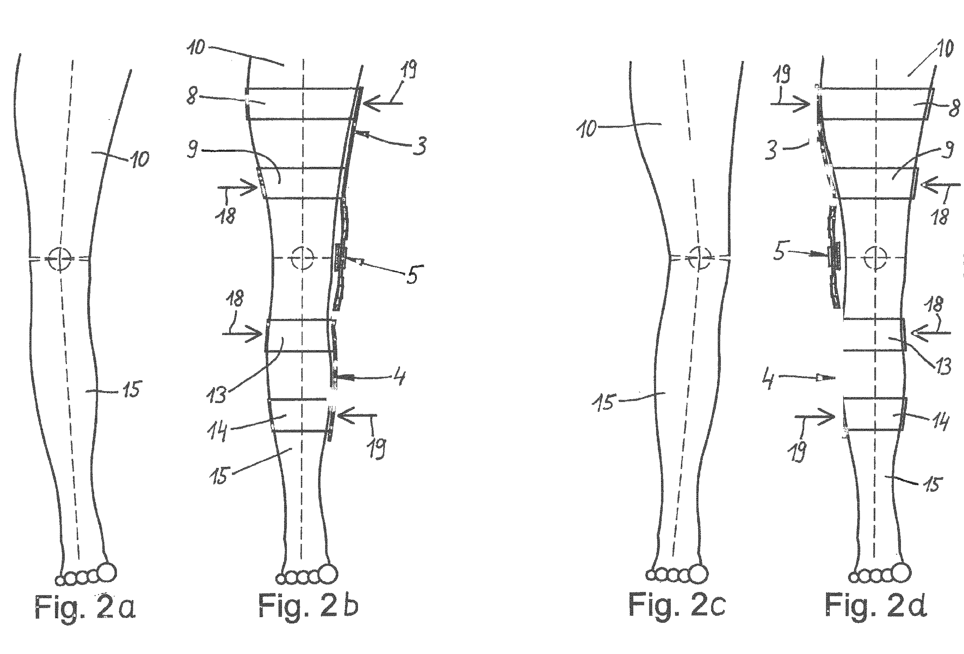

Wie aus den Figuren 2a bis 2d ersichtlich, die eine Ansicht eines Beins von vorne zeigen, kann die Orthese auf der medialen Seite des Beins (Figur 2b) oder auf der lateralen Seite des Beins (Figur 2d) angeordnet werden. Die mediale Anordnung gemäß Figur 2b wird angewendet, wenn ein O-Bein vorliegt, d.h. wenn das mediale Kompartment des Knies entlastet werden soll. Die laterale Anordnung der Orthese gemäß Figur 2d wird angewendet, wenn ein X-Bein vorliegt und das laterale Kompartment des Kniegelenks entlastet werden soll.

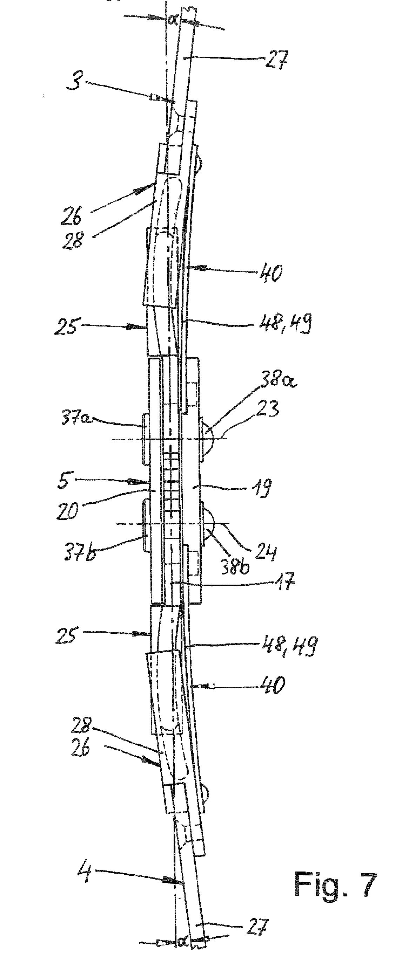

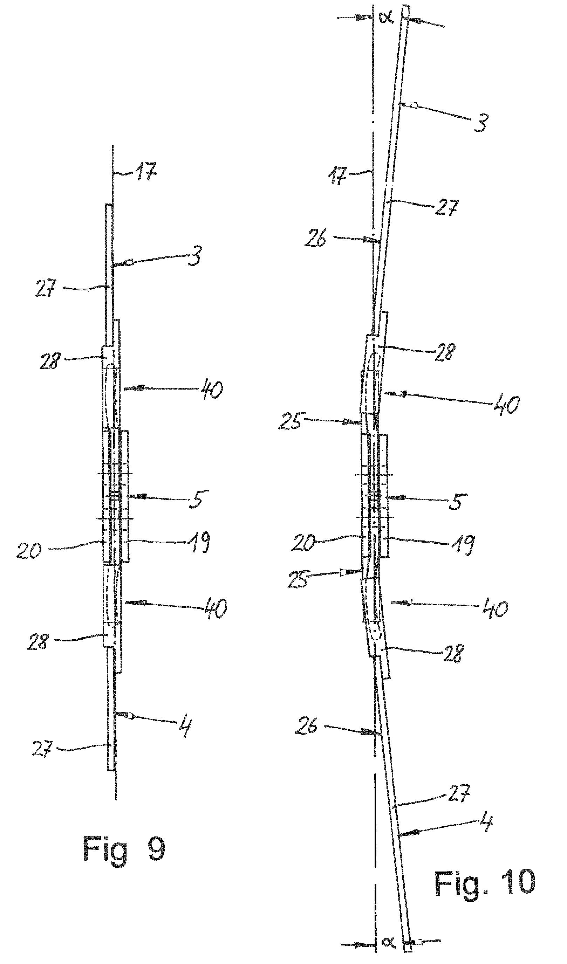

Die Figuren 7 und 10 zeigen, dass der erste Schienenarm 3 und zweite Schienenarm 4 in der Extensionsstellung relativ zu einer Hauptebene 17 der Gelenkeinrichtung 5 um einen bestimmten Inklinationswinkel α in medialer oder lateraler Richtung geneigt sein kann. Das proximale Ende des ersten (oberen) Schienenarms 3 sowie das distale Ende des zweiten (unteren) Schienenarms 4 weisen in diesem Fall zur Hauptebene 17 einen größeren Abstand auf als das distale Ende des ersten Schienenarms 3 bzw. das proximale Ende des zweiten Schienenarms 4. Bei einer Anordnung der Orthese gemäß den Figuren 2b und 2d liegt das proximale Ende des ersten Schienenarms 3 im Bereich des proximalen Haltebands 8 am Oberschenkel 10 an, während der erste Schienenarm 3 im Bereich des distalen Haltebands 9 zumindest so lange, wie das distale Halteband 9 nicht festgezogen wird, zum Oberschenkel 10 einen bestimmten Abstand aufweist. In gleicher Weise liegt das distale Ende des zweiten (unteren) Schienenarms 4 im Bereich des distalen Haltegurts 14 am Unterschenkel 15 an, während der zweite Schienenarm 4 im Bereich des proximalen Haltebands 13 zumindest so lange, wie das proximale Halteband 13 nicht festgezogen wird, zum Unterschenkel 15 einen bestimmten Abstand aufweist. Werden nun die dem Kniegelenk näheren Haltebänder 9, 13 festgezogen, werden auf den Oberschenkel 10 und Unterschenkel 15 Kräfte in Richtung der Pfeile 18 und im Bereich der Haltebänder 8, 14 entgegengesetzt gerichtete Kräfte in Richtung der Pfeile 19 aufgebracht, welche das Kniegelenk in medialer Richtung (Figur 2b) bzw. lateraler Richtung (Figur 2d) drängen. Dies bewirkt eine entsprechende Entlastung des medialen Kniegelenkkompartments (Figur 2b) bzw. lateralen Kniegelenkkompartments (Figur 2d).

Der in den Figuren 7 und 10 dargestellte mediale oder laterale Inklinationswinkel α, d.h. die Winkelstellung der ersten und zweiten Schienenarme 3, 4 im Bereich der Haltebänder 8, 9, 13, 14 relativ zur Hauptebene 17 der Gelenkeinrichtung 5, ist zweckmäßigerweise nur dann gegeben, wenn das Bein gestreckt ist, d.h. wenn die ersten und zweiten Schienenarme 3, 4 zueinander einen Winkel von 180° oder von nahe 180°, insbesondere von 160° bis 180°, aufweisen. Dieser Inklinationswinkel α, der auch als erster Inklinationswinkel bezeichnet werden kann, kann beispielsweise 2° bis 10°, vorzugsweise 3° bis 7°, betragen. Andere erste Inklinationswinkel α sind möglich. Wird das Knie gebeugt, d.h. die Orthese in Flexionsrichtung geschwenkt, verändert sich gleichzeitig der Inklinationswinkel α bis zu einem zweiten Inklinationswinkel, der sich vom ersten Inklinationswinkel unterscheidet und beispielsweise 0° bis 2°, insbesondere 0°, relativ zur Hauptebene 17 betragen kann. Dieser zweite Inklinationswinkel stellt sich vorzugsweise dann ein, wenn das Knie stark gebeugt ist, d.h. wenn der Winkel zwischen erstem und zweitem Schienenarm 3, 4 90° oder weniger beträgt. In diesem gebeugten Zustand werden die von der Orthese über die Haltebänder 8, 9, 13, 14 auf das Bein ausgeübten medialen und lateralen Zug- und Druckkräfte erheblich reduziert, wodurch die Durchblutung des Beins in diesen Bereichen gefördert und der Tragekomfort erheblich verbessert wird.

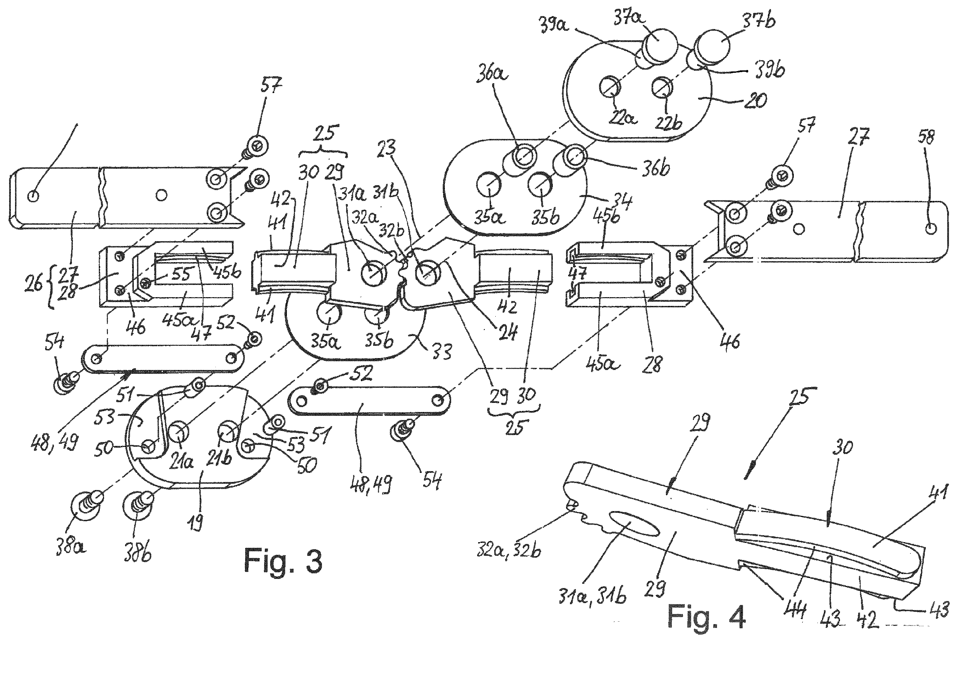

Der Aufbau der erfindungsgemäßen Orthese, welcher die automatische Veränderung des Inklinationswinkels α bei gleichzeitiger Längenveränderung der Schienenarme 3, 4 in Abhängigkeit des Flexions- bzw. Extensionswinkels der Schienenarme 3, 4 ermöglicht, wird im Folgenden anhand der Figuren 3 bis 8 näher beschrieben. Die Manschetten 1, 2 sowie Polstermaterialien sind in diesen Figuren der Übersichtlichkeit halber weggelassen.

Figur 3 zeigt die erfindungsgemäße Orthese in Explosionsdarstellung. Die Gelenkeinrichtung 5 zur schwenkbaren Lagerung der beiden Schienenarme 3, 4 umfasst eine erste Trägerplatte 19 und eine hierzu parallele zweite Trägerplatte 20. Beide Trägerplatten 19, 20 sind oval ausgebildet und weisen die gleiche Umfangskontur auf. In den Trägerplatten 19, 20 sind Bohrungen 21a, 21b bzw. 22a, 22b vorgesehen. Die Bohrungen 21a, 22a fluchten miteinander und weisen eine gemeinsame Mittelachse auf, die eine erste Schwenkachse 23 für den ersten Schienenarm 3 bildet. Die Bohrungen 21b, 22b fluchten ebenfalls miteinander und weisen eine gemeinsame Mittelachse auf, die eine zweite Schwenkachse 24 für den zweiten Schienenarm 4 bildet. Die beiden Schwenkachsen 23, 24 liegen parallel zueinander. Die Trägerplatten 19, 20 sind somit Teil der zentralen Gelenkeinrichtung 5, mit welcher die Schienenarme 3, 4 biaxial schwenkbar gelagert sind, um in Flexions- oder Extensionsrichtung verschwenkt werden zu können.

Die Schienenarme 3, 4 umfassen jeweils einen ersten Schienenarmabschnitt 25, der schwenkbar an den Trägerplatten 19, 20 gelagert ist, sowie einen zweiten Schienenarmabschnitt 26, der einen Schienenbefestigungsabschnitt 27 und einen Endabschnitt 28 umfasst und mittels des Endabschnitts 28 längsverschiebbar am ersten Schienenarmabschnitt 25 gelagert ist.

Wie insbesondere aus Figur 3 ersichtlich, sind die Endabschnitte 28 der zweiten Schienenarmabschnitte 26 getrennt von den zugeordneten Schienenbefestigungsabschnitten 27 ausgebildet und mittels Schrauben 57 an den zugeordneten Schienenbefestigungsabschnitten 27 festgeschraubt. Alternativ hierzu ist es jedoch auch möglich, Endabschnitte 28 und Schienenbefestigungsabschnitte 27 einteilig auszubilden.

Die Schienenbefestigungsabschnitte 27 weisen Bohrungen 58 auf, die zum Hindurchführen derjenigen Befestigungsmittel, insbesondere der Nieten 7, 12, dienen, mit denen die Schienenbefestigungsabschnitte 27 an den Manschettenschalen 6, 11 befestigt sind.

Die ersten Schienenarmabschnitte 25 umfassen jeweils einen plattenförmigen Lagerabschnitt 29 und einen daran anschließenden, sich in Richtung des zweiten Schienenarmabschnitts 26 erstreckenden Führungsabschnitt 30. Im gezeigten Ausführungsbeispiel sind Lagerabschnitt 29 und Führungsabschnitt 30 einteilig ausgebildet. Alternativ ist es jedoch auch möglich, den Lagerabschnitt 29 und den Führungsabschnitt 30 zweiteilig auszubilden und mittels geeigneter Befestigungsmittel fest miteinander zu verbinden.

Der plattenartige Lagerabschnitt 29 jedes ersten Schienenarmabschnitts 25 weist eine Bohrung 31a bzw. 31b auf, die zur Schwenkachse 23 bzw. 24 fluchtet. Die Schwenkwinkel der ersten Schienenarmabschnitte 25 relativ zu den Trägerplatten 19, 20 sind durch Verzahnungen 32a, 32b aufeinander abgestimmt, die an gegenüberliegenden Stirnflächen der Lagerabschnitte 29 vorgesehen sind und ineinander greifen.

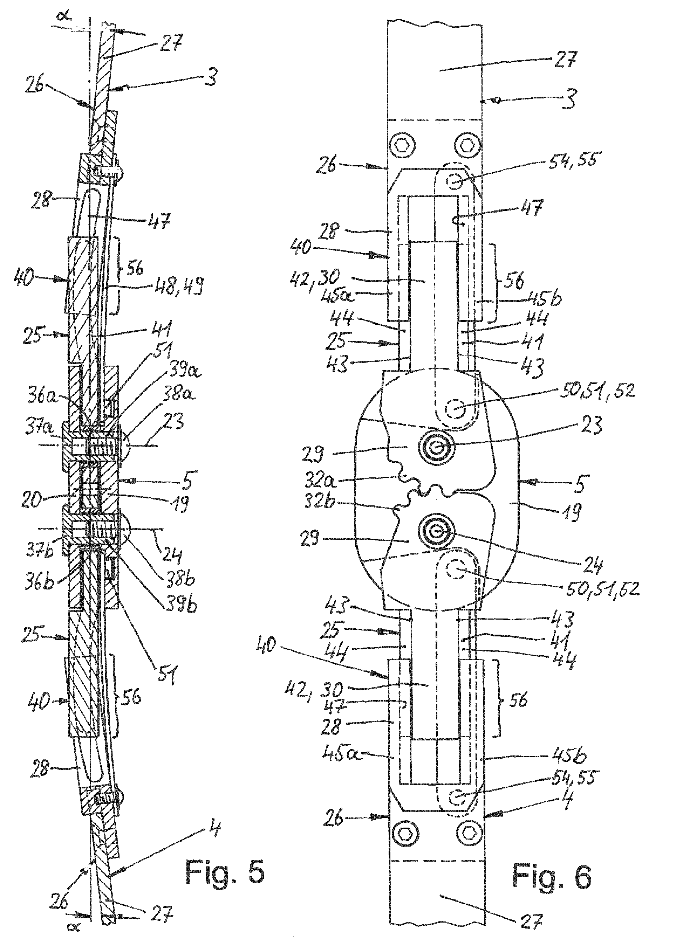

Wie auch aus den Figuren 5 und 6 ersichtlich, ist die Dicke der plattenartigen Lagerabschnitte 29 so bemessen, dass sie im Zwischenraum zwischen den parallel angeordneten Trägerplatten 19, 20 im Wesentlichen spielfrei angeordnet sind. Die Führungsabschnitte 30 befinden sich dagegen außerhalb der Trägerplatten 19, 20 und können daher, wie gezeigt, eine größere Dicke als die Lagerabschnitte 29 haben.

Zur Reibungsverminderung sind beidseits der Lagerabschnitte 29 zwischen diesen und den Trägerplatten 19, 20 Reibungsverminderungsscheiben 33, 34 vorgesehen, welche im gezeigten Ausführungsbeispiel die gleiche ovale Kontur wie die Trägerplatten 19, 20 haben. In den Reibungsverminderungsscheiben 33, 34 sind wiederum jeweils zwei Bohrungen 35a, 35b fluchtend zu den Schwenkachsen 23, 24 angeordnet. Das Material der Reibungsverminderungsscheiben 33, 34 ist bevorzugt ein reibungsarmer Kunststoff, beispielsweise Teflon.

Die Lagerung der ersten Schienenarmabschnitte 25 an den Trägerplatten 19, 20 erfolgt mittels zylindrischer innerer Lagerhülsen 36a, 36b sowie äußerer Gewindehülsen 37a, 37b und Schrauben 38a, 38b. Die Lagerhülsen 36a, 36b erstrecken sich durch die Bohrungen 35a, 31a bzw. 35b, 31b der Reibungsverminderungsscheiben 33, 34 und Lagerabschnitte 29 hindurch. Der Außendurchmesser der Lagerhülsen 36a, 36b ist dabei derart auf den Durchmesser dieser Bohrungen abgestimmt, dass eine sehr exakte radiale Führung der Lagerabschnitte 29 gegeben ist. Die Länge der Lagerhülsen 36a, 36b bestimmt den gegenseitigen Abstand der Trägerplatten 19, 20.

Die Gewindehülsen 37a, 37b weisen jeweils einen Hohlzylinderabschnitt 39a, 39b mit verringertem Außendurchmesser sowie einen größeren Kopfabschnitt auf. Die Hohlzylinderabschnitte 39a, 39b erstrecken sich im Wesentlichen spielfrei durch die Bohrungen 22a, 22b der zweiten Trägerplatte 20, die inneren Durchgangsbohrungen der Lagerhülsen 36a, 36b, sowie zumindest teilweise durch die Bohrungen 21a, 21b der ersten Trägerplatte 19 hindurch, wie auch aus Figur 5 ersichtlich ist. Die Hohlzylinderabschnitte 39a, 39b sind weiterhin mit einem Innengewinde versehen, in welches die Schrauben 38a, 38b von der gegenüberliegenden Seite der Gelenkeinrichtung 5 her derart eingeschraubt werden können, dass die beiden Trägerplatten 19, 20 fest aneinander gehalten werden. Hierbei dienen, wie bereits ausgeführt, die Lagerhülsen 36a, 36b als Abstandshalter für die Trägerplatten 19, 20.

Durch eine derartige Konstruktion ist es möglich, die ersten Schienenarmabschnitte 25 der Schienenarme 3, 4 derart schwenkbar miteinander zu verbinden, dass ein Verschwenken in Flexions- und Extensionsrichtung der ersten Schienenarmabschnitte 25 innerhalb eines vorgegebenen Schwenkbereiches möglich ist, während sie in medialer und lateraler Richtung durch die Trägerplatten 19, 20 und Reibungsverminderungsscheiben 33, 34 nicht oder kaum bewegbar sind.

Die mediale oder laterale Verschwenkung des zweiten Schienenarmabschnitts 26 relativ zum ersten Schienenarmabschnitt 25 erfolgt mittels einer Inklinationsverstelleinrichtung 40, welche zwischen dem Endabschnitt 28 der zweiten Schienenarmabschnitte 26 und dem Führungsabschnitt 30 der ersten Schienenarmabschnitte 25 wirkt und mit der Gelenkeinrichtung 5 derart zwangsgekoppelt ist, dass ein Verschwenken der ersten und zweiten Schienenarme 3, 4 in Flexions- oder Extensionsrichtung mit einer Veränderung des Inklinationswinkels α in medialer oder lateraler Richtung relativ zur Hauptebene 17 der Gelenkeinrichtung 5 verbunden ist. Als Hauptebene 17 wird hierbei eine Ebene verstanden, in welcher die Lagerabschnitte 29 der ersten Schienenarmabschnitte 25 liegen und die damit parallel zu den Hauptebenen der Trägerplatten 19, 20 liegt.

Die Inklinationsverstelleinrichtung 40 umfasst im dargestellten Ausführungsbeispiel eine Kulissenführung, mit welcher der zweite Schienenarmabschnitt 26 eines jeden Schienenarms 3, 4 in seiner Längsrichtung verschiebbar am ersten Schienenarmabschnitt 25 geführt ist.

Hierzu weisen die Führungsabschnitte 30 der ersten Schienenarmabschnitte 25 jeweils zwei gegenüberliegende Führungsstege 41 auf, die in Längsrichtung der Führungsabschnitte 30 verlaufen und, wie insbesondere aus Figur 4 ersichtlich, in medialer oder lateraler Richtung gebogen ausgeführt sind. Der Bogen liegt somit in einer Ebene, die rechtwinklig zur Hauptebene 17 des ersten Schienenarmabschnitts 25 angeordnet ist. Die Führungsstege 41 haben eine konstante Höhe, mit der sie auf gegenüberliegenden Seiten eines quaderförmigen Fortsatzes 42 der Lagerabschnitte 29 über ebene Grundflächen 43 des Fortsatzes 42 vorstehen. Hierdurch werden an den Führungsstegen 41 kreisbogenförmig gebogene Seitenführungsflächen 44 gebildet.

Die zweiten Schienenarmabschnitte 26 weisen an ihren Endabschnitten 28 Kulissenführungen auf, die entsprechend der Form der Führungsstege 41 ausgebildet sind, so dass die Endabschnitte 28 und damit die gesamten zweiten Schienenarmabschnitte 26 durch die ebenen Grundflächen 43 und die gebogenen Seitenführungsflächen 44 der Führungsstege 41 geführt sind. Die Endabschnitte 28 sind hierzu U-förmig ausgebildet und weisen gegenüberliegende Führungsschenkel 45a, 45b auf, die sich parallel zueinander von einem Verbindungsabschnitt 46 aus erstrecken. In den Führungsschenkeln 45a, 45b befindet sich jeweils eine gebogene Führungskulisse 47, in welcher ein Führungssteg 41 des ersten Schienenarmabschnitts 25 längs verschiebbar geführt ist. Weiterhin liegen die Innenflächen der Führungsschenkel 45a, 45b an den Grundflächen 43 des Fortsatzes 42 an. Werden die Endstücke 28 längs des Führungsabschnitts 30 der ersten Schienenarmabschnitte 25 verschoben, führt das Endstück 28 und damit der gesamte zweiten Schienenarmabschnitt 26 eine bogenförmige Bewegungskurve aus, wodurch der zweite Schienenarmabschnitt 26 in medialer oder lateraler Richtung relativ zum ersten Schienenarmabschnitt 25 verschwenkt wird.

Die Längsverschiebung des zweiten Schienenarmabschnitts 26 relativ zum ersten Schienenarmabschnitt 25 erfolgt mittels einer Verschiebeeinrichtung 48, die ein Verbindungselement in der Form einer Verbindungsstrebe 49 umfasst. Diese Verbindungsstrebe 49 ist an einem ersten Ende an der ersten Trägerplatte 19 der Gelenkeinrichtung 5 befestigt. Hierzu weist die erste Trägerplatte 19, wie aus Figur 3 ersichtlich, eine Bohrung 50 auf, in der eine Hülse 51 mit Innengewinde gelagert ist. An dieser Hülse 51 ist die Verbindungsstrebe 49 mittels einer Schraube 52 befestigbar, die durch eine entsprechende Bohrung der Verbindungsstrebe 49 hindurchgeführt und in das Innengewinde der Hülse 51 eingeschraubt wird. Die Hülse 51 ist in der Bohrung 50 drehbar gelagert, so dass die Verbindungsstrebe 49 relativ zur Trägerplatte 19 verschwenkt werden kann.

Damit die Verbindungsstrebe 49 zwischen den beiden Trägerplatten 19, 20 Platz findet, weist die Trägerplatte 19 zwei vertiefte Taschen, d.h. Aussparungen 53, auf, die sich über den möglichen Schwenkbereich der Verbindungsstreben 49 erstrecken. Die Verbindungsstreben 49 erstrecken sich in den Bereich dieser Aussparungen 43 derart hinein, dass sie quer zur Trägerplatte 19 nicht über die innere Seitenfläche der Trägerplatte 19 vorstehen.

Die Verbindungsstreben 49 sind weiterhin in ihrem zweiten, gegenüberliegenden Endbereich mit den zweiten Schienenarmabschnitten 26 schwenkbar gekoppelt. Dies erfolgt über Schrauben 54, die durch eine Bohrung der Verbindungsstreben 49 hindurchgeführt und in eine Gewindebohrung 55 der Endabschnitte 28 der zweiten Schienenarmabschnitte 26 eingeschraubt werden.

Bohrung 50, Hülse 51 und Schraube 52 bilden damit eine erste oder innere Lagerstelle zur Kopplung der Verbindungsstreben 49 mit der Gelenkeinrichtung 5, während Schraube 54 und Gewindebohrung 55 eine zweite oder äußere Lagerstelle zur Kopplung der Verbindungsstreben 49 mit den zweiten Schienenarmabschnitten 26 bilden. Die erste Lagerstelle ist dabei derart versetzt zu den jeweiligen Schwenkachsen 23, 24 angeordnet, dass, wenn die Schienenarme 3, 4 von ihrer maximal gestreckten Stellung (Extensionsstellung) in ihre maximal abgewinkelte Stellung (Flexionsstellung) überführt werden, die zweite oder äußere Lagerstelle aufgrund der Verbindungsstreben 49 einen Kreis beschreibt, der sich den Schwenkachsen 23 bzw. 24 kontinuierlich nähert. Dies bedeutet, dass die zweiten Schienenarmabschnitte 26 bei einer Bewegung von der Extensionsstellung in die Flexionsstellung mittels der Verbindungsstreben 49 kontinuierlich in Richtung der Trägerplatten 19, 20 verschoben werden, wodurch sich die Schiebearme 3, 4 verkürzen. Hierbei gleiten die Endabschnitte 28 längs der Führungsabschnitte 30 der ersten Schienenarmabschnitte 25 und werden dabei gleichzeitig entsprechend der bogenförmigen Kontur der Führungskulissen 47 und Führungsstege 41 in medialer oder lateraler Richtung verschwenkt.

Umgekehrt werden die zweiten Schienenarmabschnitte 26 in ihrer Längsrichtung von den Schwenkachsen 23 bzw. 24 und damit von den Lagerabschnitten 29 weggeschoben, d.h. entfernt, wenn die Schienenarme 3, 4 von ihrer maximalen Flexionsstellung in ihre Extensionsstellung geschwenkt werden. Die Schiebearme 3, 4 verlängern sich dabei entsprechend.

Diese Verlängerung der Schienenarme 3, 4 bewirkt im dargestellten Ausführungsbeispiel, dass die obere Manschette 1 in proximale Richtung und die unteren Manschette 2 in distale Richtung bewegt werden, wenn die Orthese von ihrer Flexionsstellung in ihre Extensionsstellung überführt wird. Hierdurch wird das femorotibiale Kompartment auseinandergezogen.

Die Figuren 5 bis 7 zeigen die Orthese in ihrer maximalen Extensionsstellung, in der die Schienenarme 3, 4 in der Seitenansicht, die in Figur 6 gezeigt ist, in einem Winkel von 180° zueinander angeordnet sind. Wie insbesondere aus Figur 6 ersichtlich, überlappen sich in dieser Stellung die Endabschnitte 28 der zweiten Schienenarmabschnitte 26 und die Führungsabschnitte 30 der ersten Schienenarmabschnitte 25 in einem Überlappungsbereich 56. Dieser ist ausreichend groß, um eine exakte Führung der zweiten Schienenarmabschnitte 26 an den ersten Schienenarmabschnitten 25 nach allen Seiten hin zu gewährleisten. Wie aus Figur 5 ersichtlich, verlaufen in diesem Überlappungsbereich 56 die Führungskulissen 47 und Führungsstege 41 derart, dass die zweiten Schienenarmabschnitte 26 relativ zur Hauptebene 17 der Gelenkeinrichtung 5 in medialer oder lateraler Richtung schräg geneigt sind und einen Inklinationswinkel α aufweisen, der im gezeigten Ausführungsbeispiel etwa 5° beträgt.

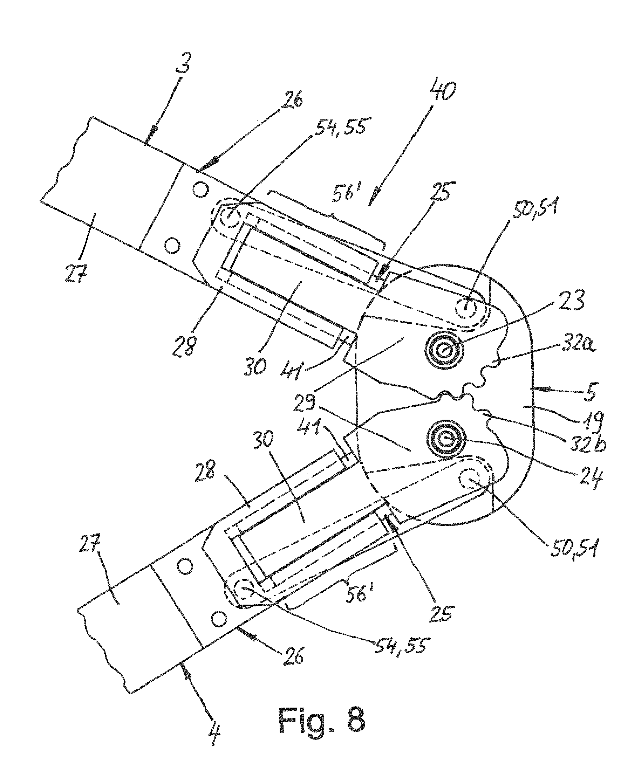

In den Figuren 8 und 9 ist eine Stellung der Orthese gezeigt, in welcher die Schienenarme 3, 4 zueinander maximal gebeugt sind, d.h. einen minimalen Flexionswinkel zueinander einnehmen. Dieser beträgt im gezeigten Ausführungsbeispiel etwa 60°. Wie ersichtlich, befinden sich in dieser Stellung die zweiten Schienenarmabschnitte 26 aufgrund der Positionssteuerung durch die Verbindungsstreben 49 näher an den jeweiligen Lagerabschnitten 29 bzw. Schwenkachsen 23, 24. Der Überlappungsbereich 56' ist entsprechend vergrößert. Führungskulissen 47 und Führungsstege 41 sind derart ausgebildet, dass in dieser Stellung der mediale oder laterale Inklinationswinkel α der zweiten Schienenarmabschnitte 26 reduziert ist und im vorliegenden Ausführungsbeispiel 0° beträgt, wie aus Figur 9 ersichtlich.

Im Rahmen der Erfindung ist eine Vielzahl von Variationen möglich. Beispielsweise ist es grundsätzlich möglich, die Inklinationsverstelleinrichtung 40 und Schienenarm-Längenveränderungseinrichtung nicht an beiden Schienenarmen 3, 4 gleich, sondern unterschiedlich derart auszubilden, dass sich bei einer Änderung des Flexions- oder Extensionswinkels der Schienenarme 3, 4 der mediale oder laterale Inklinationswinkel α und/oder die Länge des ersten Schienenarms 3 anders ändert als derjenige bzw. diejenige des zweiten Schienenarms 4. Weiterhin ist es auch möglich, die Inklinationsverstelleinrichtung 40 und Schienenarm-Längenveränderungseinrichtung nur an einem der Schienenarme 3, 4 vorzusehen. Weiterhin ist die Erfindung nicht nur bei Orthesen mit Biaxialgelenken, sondern auch bei solchen einsetzbar, die nur eine einzige Schwenkachse aufweisen. Die Führungskulissen 47 und Führungsstege 41 müssen nicht zwangsläufig kreisbogenförmig gekrümmt sein, sondern können auch andere Krümmungen haben. Die Endabschnitte 28 können auch einstückig mit den Schienenbefestigungsabschnitten 27 ausgebildet sein. Während gemäß den Figuren 2a und 2b die erfindungsgemäße Orthese lediglich auf der medialen oder lateralen Seite eines Körpergelenks angeordnet ist, ist es auch möglich, auf der gegenüberliegenden Seite zusätzlich eine Orthese ohne Inklinationsverstelleinrichtung oder eine erfindungsgemäße Orthese derart anzuordnen, dass die Entlastungsbewegung für das geschädigte Gelenkkompartment unterstützt wird. Weiterhin können die Führungskulissen 47 auch an den ersten, schwenkbar gelagerten Schienenarmabschnitten 25 und die damit zusammenwirkenden Führungselemente an den zweiten Schienenarmabschnitten 26 vorgesehen sein.

## Patentansprüche

1. Orthese, insbesondere Knieorthese, die umfasst:
- ein Paar Schienenarme (3, 4), die jeweils einen schwenkbar gelagerten ersten Schienenarmabschnitt (25) und einen an dem Körper eines Patienten befestigbaren zweiten Schienenarmabschnitt (26) aufweisen,
- eine Gelenkeinrichtung (5) zum Verschwenken der Schienenarme (3, 4) in Flexions- und Extensionsrichtung, und
- eine Inklinationsverstelleinrichtung (40) zum Verstellen der medialen oder lateralen Inklination des zweiten Schienenarmabschnitts (26) zumindest eines Schienenarms (3, 4) relativ zur Gelenkeinrichtung (5),
- wobei der zweite Schienenarmabschnitt (26) zumindest eines Schienenarms (3, 4) derart bewegbar am ersten Schienenarmabschnitt (25) geführt und mit der Gelenkeinrichtung (5) gekoppelt ist, dass ein Verschwenken der Schienenarme (3, 4) in Flexions- oder Extensionsrichtung mit einer Verstellung der medialen oder lateralen Inklination des zweiten Schienenarmabschnitts (26) zwangsgekoppelt ist,
**dadurch gekennzeichnet, dass** der zweite Schienenarmabschnitt (26) zumindest eines Schienenarms (3, 4) derart neigungsverstellbar und in Längsrichtung des Schienenarms (3, 4) verschiebbar am ersten Schienenarmabschnitt (25) gehaltert und zwangsgeführt ist, dass ein Verschwenken des Schienenarms (3, 4) in Flexions- oder Extensionsrichtung sowohl mit einer Verstellung der medialen oder lateralen Inklination des zweiten Schienenarmabschnitts (26) als auch mit einer Längenänderung des Schienenarms (3, 4) gekoppelt ist.

2. Orthese nach Anspruch 1, **dadurch gekennzeichnet, dass** die Inklinationsverstelleinrichtung (40) eine Kulissenführung umfasst, mit welcher der zweite Schienenarmabschnitt (26) verschiebbar am ersten Schienenarmabschnitt (25) geführt ist.

3. Orthese nach Anspruch 2, **dadurch gekennzeichnet, dass** die Kulissenführung mindestens eine am ersten oder zweiten Schienenarmabschnitt (25, 26) angeordnete, in medialer oder lateraler Richtung gebogene und/oder schräg verlaufende Führungskulisse (47) und mindestens ein am anderen Schienenarmabschnitt (26, 25) angeordnetes, in die Führungskulisse (47) eingreifendes Führungselement umfasst.

4. Orthese nach Anspruch 3, **dadurch gekennzeichnet, dass** der erste oder zweite Schienenarmabschnitt (25, 26) einen U-förmigen Endabschnitt (28) mit gegenüberliegenden Führungsschenkeln (45a, 45b) aufweist, in denen jeweils eine Führungskulisse (47) angeordnet ist, und dass der andere Schienenarmabschnitt (26, 25) einen zwischen die Führungsschenkel (45a, 45b) einführbaren Führungsabschnitt (30) mit auf gegenüberliegenden Seiten des Führungsabschnitts (30) angeordneten Führungselementen aufweist.

5. Orthese nach Anspruch 3 oder 4, **dadurch gekennzeichnet, dass** das Führungselement als länglicher, an die Form der Führungskulisse (47) angepasster Führungssteg (41) ausgebildet ist.

6. Orthese nach einem der vorhergehenden Ansprüche, **dadurch gekennzeichnet, dass** die Gelenkeinrichtung (5) mindestens eine Trägerplatte (19, 20) aufweist, an welcher der erste Schienenarmabschnitt (25) um mindestens eine Schwenkachse (23, 24) schwenkbar gelagert ist, und dass eine Verschiebeeinrichtung (48) vorgesehen ist, die einerseits mit der Trägerplatte (19, 20) und andererseits mit dem zweiten Schienenarmabschnitt (26) mindestens eines Schienenarms (3, 4) derart wirkverbunden ist, dass ein Verschwenken des Schienenarms (3, 4) in Flexions- oder Extensionsrichtung sowohl mit einer Verstellung des zweiten Schienenarmabschnitts (26) in medialer oder lateraler Richtung als auch mit einem Entfernen oder Annähern des zweiten Schienenarmabschnitts (26) von der bzw. an die Schwenkachse (23, 24) zwangsgekoppelt ist.

7. Orthese nach Anspruch 6, **dadurch gekennzeichnet, dass** die Verschiebeeinrichtung (48) mindestens eine Verbindungsstrebe (49) umfasst, die einerseits mit Abstand zur Schwenkachse (23, 24) mit der Trägerplatte (19, 20) und andererseits mit dem zweiten Schienenarmabschnitt (26) mindestens eines Schienenarms (3, 4) verbunden ist, derart, dass sich bei einem Verschwenken des Schienenarms (3, 4) in Flexions- oder Extensionsrichtung der zweite Schienenarmabschnitt (26) relativ zum ersten Schienenarmabschnitt (25) verschiebt.

8. Orthese nach einem der vorhergehenden Ansprüche, **dadurch gekennzeichnet, dass** die Inklinationsverstelleinrichtung (40) derart ausgebildet ist, dass der zweite Schienenarmabschnitt (26) zumindest eines Schienenarms (3, 4) in einer Flexionsstellung der Schienenarme (3, 4) von bis zu 90° in einer Hauptebene (17) der Gelenkeinrichtung (5) liegt oder zumindest weitgehend parallel hierzu angeordnet ist, während sich der zweite Schienenarmabschnitt (26) bei Überführung der Schienenarme (3, 4) aus dieser Flexionsstellung in Richtung einer 180° Stellung zunehmend in die mediale oder laterale Richtung neigt.
